# EUROPEAN PATENT APPLICATION

(11) **EP 4 678 118 A1**
(43) Date of publication of application: **14.01.2026**
(21) Application number: 25183739.9
(22) Date of filing: 18.06.2025
(51) Int. Cl.: A61B 10/06, A61B 17/29, A61B 10/04, A61B 10/02

(54) **SURGICAL DEVICE FOR BIOPSY**

(30) Priority: 12.07.2024 IT 202400016240
(71) Applicant: THD S.p.A., 42015 Correggio (RE) (IT)
(72) Inventor: BASTIA, Filippo, 41019 Soliera (IT)
(74) Representative: Paparo, Aldo

(57) **Abstract**

A surgical device (1) for biopsy comprising an external structure (2) having a handle (21), which is disposed in a proximal portion (2p) of the external structure (2) and may be gripped by a user, an external element (3) insertable, during use, into a cavity, biopsy forceps (4) retained by the external element (3), an actuation mechanism (5) for the forceps (4), disposed inside the external structure (2), and a trigger (6) pivoted and solidly joined to the external structure (2) and engaged with the actuation mechanism (5). A main structure (51) of the actuation mechanism (5) comprises a pin (58) and the trigger (6) has a through hole (61) shaped like a cam and pivoted on the pin (58) of the main structure of the actuation mechanism (5).

## Description

The present invention relates to a surgical device.

The present invention has application in the field of surgical devices for biopsy.

In particular, biopsy is a medical examination consisting in the removal of a portion or fragment of tissue from a living organism. In particular, the portion or fragment of tissue removed can be analysed under a microscope or by means of other microbiology or molecular biology techniques.

There are known devices for removing the portion or fragment of tissue, such as forceps or other similar devices.

There are known forceps or other similar devices that have application in human cavities and orifices, either natural or obtained surgically.

Some of these devices have application for use together with inspection devices, such as anoscopes, proctoscopes, rectoscopes, vaginal speculums and other similar devices.

In other words, such devices could be inserted into a hollow tubular dilator body extending between a distal opening for visually and operatively accessing mucosa delimiting the cavity, and a proximal opening that may be accessed by the practitioner at the time of use. In other words, the forceps can be introduced into the proximal opening and made to exit from the distal opening in order to remove the portion or fragment of tissue.

In general, it is well known that such instruments require precise, reliable actuation mechanisms in order to avoid problems such as blocking of the actuation mechanism itself, of the forceps or of other components.

For example, there are known devices provided with a trigger wherein the use thereof may result in the application of forces on the mechanism in unwanted directions, with a consequent blocking thereof and thus of the forceps.

Furthermore, in particular in the case of use with anoscopes, rectoscopes and the like, it could be necessary to have to modify an orientation of the forceps in order to better remove the portion or fragment which, for reasons tied to the particularity of the instruments used, cannot be immediately and easily achieved.

The technical task of the present invention is thus to provide a surgical device that is capable of overcoming the limitations emerging from the prior art.

The object of the present invention is thus to provide a surgical device in which undesirable blocking of the actuation mechanism is prevented.

A further object of the present invention is thus to provide a surgical device in which it is also possible to easily adjust an orientation of the forceps. The stated technical task and specified objects are substantially achieved by a surgical device comprising the technical features disclosed in one or more of the appended claims. The dependent claims correspond to possible embodiments of the invention.

In particular, the stated technical task and specified objects are substantially achieved by a surgical device for biopsy. According to one aspect of the present invention, the surgical device defines a biopsy system together with an anoscope, proctoscope, rectoscope, vaginal speculum or other similar device.

The surgical device for biopsy comprises an external structure having a handle, which is disposed in a proximal portion of the external structure and may be gripped by a user.

The external structure defines an internal volume and has specific housings for the internal components of the surgical device.

The external structure is preferably defined by a first half shell and a second half shell defining, in an assembled configuration, an internal volume having specific housings for the internal components of the surgical device.

The surgical device further comprises an external element extending along a main axis of extension between a first end, connected to a distal portion of the external structure, and a second end.

According to one aspect of the present invention, the external element is insertable, during use, into a cavity.

According to one aspect of the present invention, the external element is rotatable about its main axis of extension.

The surgical device further comprises biopsy forceps retained by the second end of the external element. Preferably, during use the biopsy forceps are rotatable together with the external element about the main axis of extension of the external element.

The surgical device further comprises an actuation mechanism for the forceps, disposed inside the external structure. In other words, the actuation mechanism is contained at least partly in one or more of the housings defined by the external structure.

According to one aspect of the present invention, the actuation mechanism comprises a main structure movable inside the external structure between a rest position in which the main structure is proximal to the external element and a distanced position in which the main structure is distal relative to the external element. Preferably, the external structure comprises a housing at least partly defining a guide for said main structure.

According to one aspect of the present invention, the actuation mechanism comprises an actuating wire extending at least partly inside the external element and having a first end connected to the forceps and a second end connected to a distal end of the main structure of the actuation mechanism itself. Preferably, the actuating wire extends inside the external element and parallel to the main axis of extension. Preferably, the actuating wire is free to rotate about the main axis of extension.

According to one aspect of the present invention, the actuation mechanism comprises a return spring connected to a proximal end of the main structure and configured to bring the main structure back from the distanced position to the rest position.

According to one aspect of the present invention, the actuation mechanism comprises a trigger pivoted and solidly joined to the external structure and engaged in the main structure of the actuation mechanism. The trigger is movable between a position distal to the handle and a position proximal to the handle.

In the position distal to the handle of the trigger, the main structure is in the rest position and the forceps are in an open configuration.

In the position proximal to the handle of the trigger, the main structure is in the distanced position so as to pull the wire and bring about a closed configuration of the forceps.

Preferably, the movement of the trigger from the distal position to the position proximal to the handle allows the trigger to interact with the main structure of the actuation mechanism, so as to bring about the closing and subsequent opening of the forceps.

According to one aspect of the present invention, the main structure comprises a pin and the trigger has a through hole shaped like a cam and pivoted on the pin of the main structure of the actuation mechanism. Advantageously, the cam enables the trigger to bring about a linear movement of the main structure of the actuation mechanism without transmitting forces that might cause unwanted misalignments that could block the main structure and hence the wire and the closing of the forceps. In other words, the cam of the trigger, in conjunction with the various components of the actuation mechanism, makes it possible to maintain the main structure of the actuation mechanism on an axis (during use parallel to or coinciding with the main axis of extension of the external element) so as to prevent misalignments that could cause blocking or jamming of the mechanism itself and thus of the forceps.

According to one aspect of the present invention, the latter further relates to a surgical biopsy system comprising a surgical device as described above and an anoscope, a proctoscope, a rectoscope, a vaginal speculum or another similar device having a dilator body defining at least a distal opening and a proximal opening and wherein the surgical device is insertable, during use, into the proximal opening in order to reach the distal opening and thus the cavity into which the dilator body is inserted. Additional features and advantages of the present invention will emerge more clearly from the approximate and thus non-limiting description of an embodiment of a surgical device.

The description will be set forth here below with reference to the appended drawings, provided solely by way of illustration and hence not by way of limitation, in which:

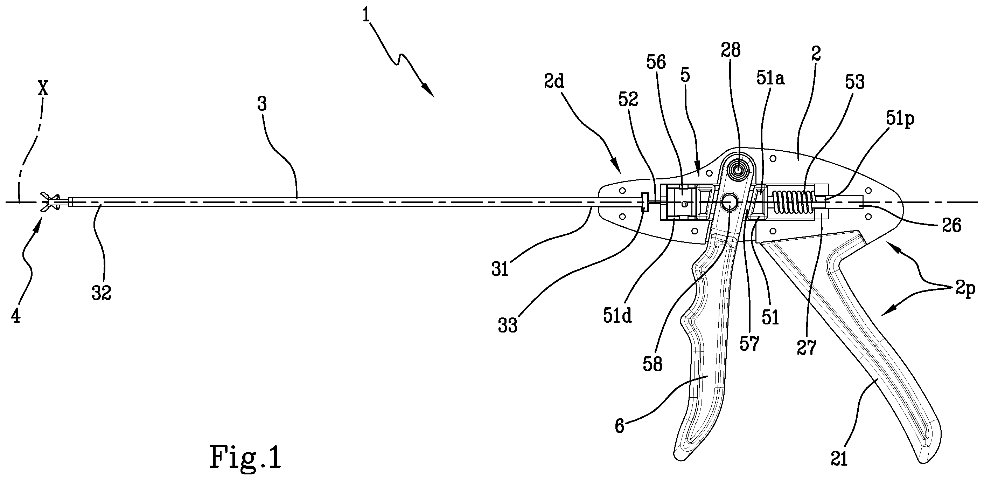

- Figure 1 is a sectional schematic representation of the surgical device of the present invention;

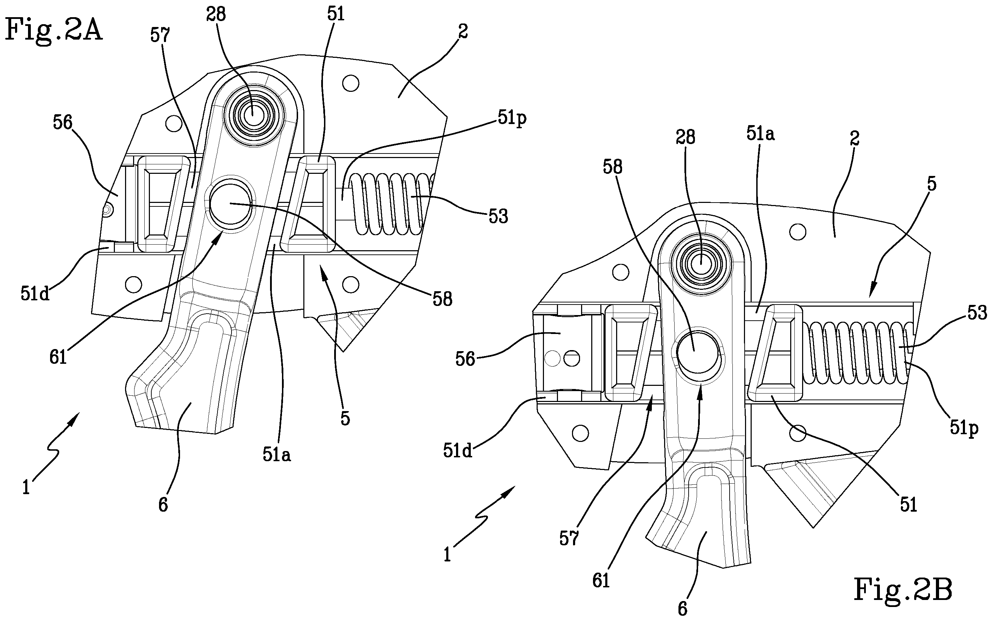

- Figures 2A and 2B are representations of the device in figure 1 according to different operating configurations;
- Figures 3A and 3B are schematic representations of details of the surgical device of the present invention;
- Figures 3C, 3D and 3E are schematic representations of details of the surgical device of the present invention according to different embodiments;
- Figure 4 is an exploded schematic representation of at least part of the components of the device of the present invention;
- Figures 5A and 5B are schematic representations of a further embodiment of the device of the present invention.

With reference to the appended figures, 1 indicates in its entirety a surgical device for biopsy which, for the sake of simplicity of description, will be indicated as the surgical device 1.

The surgical device 1, during use, may be utilised, i.e. has application in human cavities and orifices, either natural or obtained surgically.

Preferably, the surgical device 1, during use, may be utilised with an anoscope, a proctoscope, a rectoscope, a vaginal speculum or another similar device so as to define a biopsy system. In other words, the biopsy system comprises a surgical device 1, as will be described in one or more of the embodiments thereof in the present description, and an anoscope, a proctoscope, a rectoscope, a vaginal speculum or another similar device. Therefore, the surgical device 1 is insertable, during use, into a proximal opening of one of the aforesaid devices in order to reach the distal opening and thus the cavity.

The surgical device 1 comprises an external structure 2 having a handle 21, which is disposed in a proximal portion 2p of the external structure 2 itself and may be gripped by a user.

Absent any indications otherwise, in the present description the term proximal will be understood to mean any portion of the device "close" to the user and distal portion any portion of the device "far" from the user in a configuration of use of the surgical device 1.

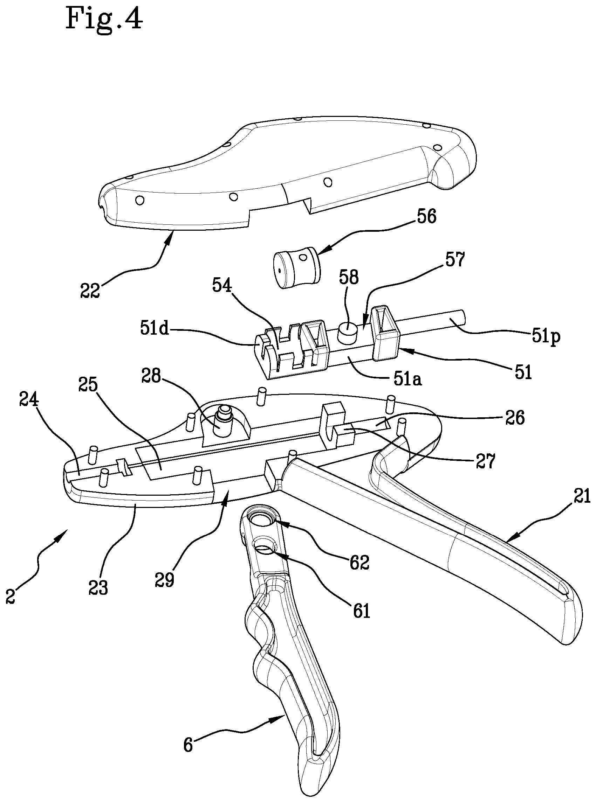

Figure 4 schematically represents an exploded view of the external structure 2 according to an embodiment thereof. In this embodiment, the external structure 2 is defined at least by a first half shell 22 and a second half shell 23.

Preferably, one of the two half shells, the half shell 23 in the appended figures, defines a plurality of seats, or housings, or guides, into which the different components making up the surgical device 1 are inserted, and which will be described in greater detail together with the respective components.

The surgical device 1 comprises an external element 3 extending along a main axis of extension "X". The external element 3 extends between a first end 31, connected to a distal portion 2d of the external structure 2, and a second end 32. The external element 3 is insertable, during use, into a cavity, i.e. inside an anoscope, a rectoscope or another similar device in order to reach the cavity.

The external element 3 is made in the form of a hollow tubular structure. Preferably, the external element 3 has an overall length of between 10 cm and 25 cm between the first end 31 and the second end 32. Other lengths for the external element 3 are possible depending on the application of the surgical device 1.

Preferably, the external element 3 has, at the first end 31, a retaining portion 33 for retaining the external element 3 itself. In other words, the retaining portion 33 is configured to make a connection between the external element 3 and the distal portion 2d of the external structure 2. Preferably, the external structure 2 comprises, in the distal portion 2d, a retaining seat 24 for the retaining portion 33.

Preferably, the retaining portion 33 and the retaining seat 24 are complementarily shaped.

Preferably, the retaining portion 33 and the retaining seat 24 are shaped in such a way as to allow a rotation of the retaining portion 33, i.e. of the external element 3, about the main axis of extension "X" of the external element 3 itself.

Preferably, the retaining portion 33 has a cylindrical or discoidal shape (or a similar shape) and the retaining seat 24 is complementarily shaped with respect to the retaining portion 33 (and thus likewise has a cylindrical or discoidal shape). In this manner, the retaining portion 33, with a cylindrical, discoidal or similar shape is free to rotate inside the retaining seat 24. The surgical device 1 further comprises biopsy forceps 4 retained by the second end 32 of the external element 3. The forceps 4 make it possible, on reaching the tissue to be analysed, to pinch the tissue of interest in order to remove the portion or fragment of tissue.

Preferably, the forceps 4 are solidly connected to/retained at the second end 32 and is therefore able to rotate together with the second end 32, i.e. the external element 3.

Advantageously, the possibility of the forceps 4, i.e. of the external element 3, to rotate on themselves, makes it possible, during use, to be able to orient the forceps, without having to assume uncomfortable positions, by directly rotating the external structure 2. In other words, in a configuration of use in which it is intended to remove a portion or a fragment of tissue located in a certain manner inside the cavity and it is necessary to adjust the forceps 4 in a certain manner, it will be possible (preferably manually) for the user to make the external element 3 rotate so as to make the forceps 4 rotate at the same time.

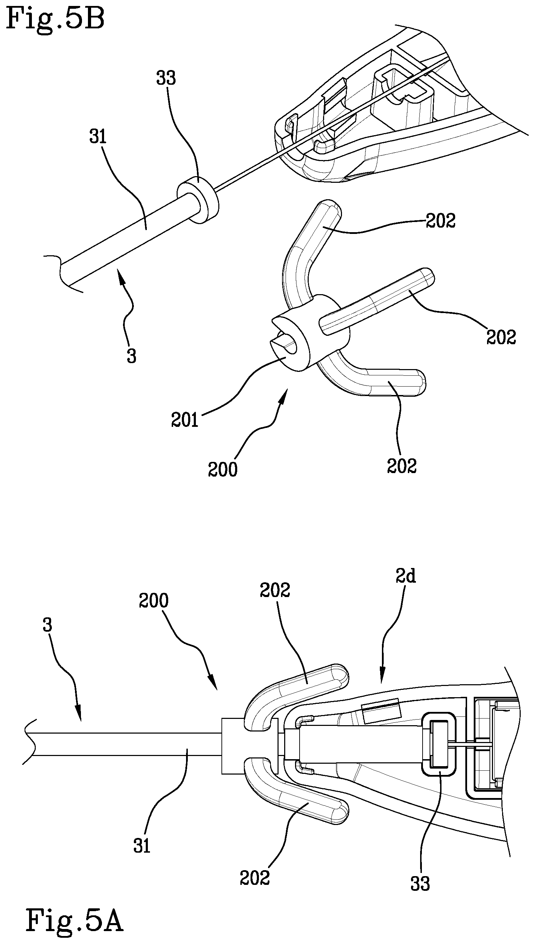

Figures 5A and 5B illustrate a further embodiment of the surgical device 1, wherein the latter comprises an actuation device 200 configured, during use, to set the external element 3, i.e. the forceps 4, into rotation.

The actuation device 200 comprises a main body 201 configured to be fitted around the external element 3. For example, the main body 201 substantially has a "C" shape defining a seat suitable for achieving a snug fit thereof around the external element 3.

In a configuration of use, the actuation device 200 is fitted in proximity to the first end 31 and to the distal portion 2d of the external structure 2. Preferably, and as represented in the appended figures, the actuation device 200 comprises a plurality of arms 202 that extend towards the distal portion 2d. In particular, the arms 202 have a shape such as to allow them to rotate about the distal portion 2d.

During use, a user who intends to set the forceps 4 into rotation can interact with the arms 202 and use them for leverage to set the external element 3 into rotation.

Preferably, the forceps 4 comprise a connecting element 41 which is disposed inside the external element 3. Preferably, the connecting element 41 is free to move inside the external element 3 itself, as will emerge more clearly below in the present description. The connecting element 41 is preferably disposed and movable inside the external element 3 in proximity to the second end 32.

Preferably, the forceps 4 comprise a grasping portion 42 defined by two arms pivoted to each other by means of a pin 43 fixed inside the external element 3. Each arm extends between a connecting end 42c and a grasping end 42a and 42b.

Preferably, the grasping ends 42a and 42b have a cup-like or spoon-like shape so that, in a closed configuration of the forceps 4 (in which the two grasping ends 42a and 42b are brought close to each other), they define a containment volume in which to retain the portion or fragment of tissue to be analysed.

Preferably, the forceps 4 comprise a pair of connecting rods 44a and 44b, each extending between a first end connected to the connecting element 41 and a second end connected to said connecting end 42c of the respective arms.

The connecting element 41 is itself movable between a first position and a second position.

In the first position, the connecting element 41 is proximal to the second end 32 of the external element 3 and the grasping portion 42 is in an open configuration wherein the grasping ends 42a and 42b are distanced from one another.

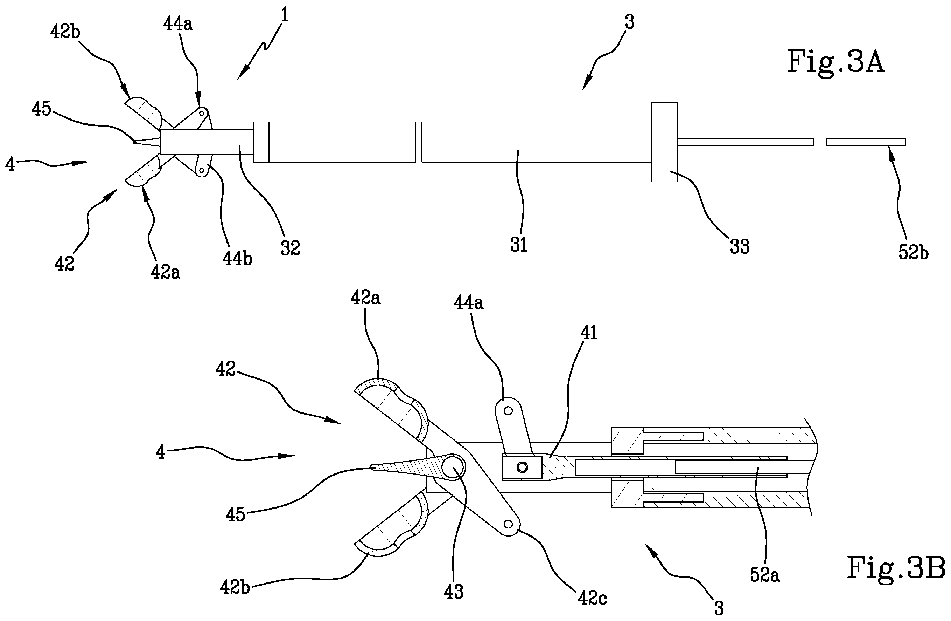

In the second position, the connecting element 41 is distanced from the second end 32 of the external element 3 so as to draw the pair of connecting rods 44a and 44b and bring the grasping ends 42a and 42b close together so as to define the closed configuration of the forceps 4. Preferably, and as represented in the appended figures (in particular in figures 3A and 3B), the forceps 4 further comprise, connected to the pin 43 of the grasping portion 42, i.e. to the second end 32 of the external element 3, a tip or needle (indicated in its entirety with the reference number 45) protruding from the second end 32 of the external element 3. Advantageously, the tip or needle 45 makes it possible to penetrate the portion or fragment of tissue so as to facilitate the removal of the portion or fragment of tissue by means of the grasping portion 42.

The surgical device 1 further comprises an actuation mechanism 5 for the forceps 4.

The actuation mechanism 5 is disposed inside the external structure 2. In particular, part of the actuation mechanism 5 is disposed inside the external structure 2 and part inside the external element 3. Therefore, the actuation mechanism 5 is disposed at least partly inside the external structure 2.

The actuation mechanism 5 comprises a main structure 51, an actuating wire 52 and a spring 53.

The main structure 51 is movable inside the external structure 2 between a rest position and a distanced position.

In the rest position, the main structure 51 is proximal, i.e. close to, the element 3 (i.e. to the distal portion 2d of the external structure 2).

In the distanced position, the main structure 51 is distal to, i.e. distanced from, the external element 3 (i.e. it is close to the proximal portion 2p of the external structure 2).

Preferably, the external structure 2 has a guide 25 in which the main structure 51 is movable. In other words, the guide 25 extends between the distal portion 2d and the proximal portion 2p of the external structure 2. The main structure 51 extends between a proximal end 51p and a distal end 51d.

Therefore, the main structure 51 has an elongate shape and has an axis of extension parallel to or coinciding with the main axis of extension "X" of the external element 3.

Preferably, the proximal end 51p of the main structure 51 is made in the form of a piston and the external structure 2 defines a sliding guide 26 for the piston.

Preferably, the distal end 51d of the main structure 51 is made in the form of a chamber 54. Preferably, the chamber 54 has a cubic shape. Preferably, the chamber 54 has a cylindrical shape.

Preferably, the main structure 51 comprises, i.e. has, a central portion 51a having at least respective distal and proximal walls.

The actuating wire 52 extends at least partly inside the external element 3.

The actuating wire 52 has a first end 52a connected to the forceps 4 and a second end 52b connected to the distal end 51d of the main structure 51. Preferably, the actuating wire 52 has a diameter of 0.5 mm.

Preferably, the actuation mechanism 5 further comprises a retaining cylinder 56, to which the second end 52b of the actuating wire 52 is connected.

The retaining cylinder 56 is disposed inside the chamber 54 (preferably cylindrical) and is free to rotate inside the chamber 54. In this manner, the actuating wire 52, too, is able to rotate freely about the main axis of extension "X" of the external element 3.

Preferably, the forceps 4, i.e. the connecting element 41, are connected to the first end 52a.

The return spring 53 is connected to the proximal end 51p of the main structure 51 and is configured to bring the main structure 51 back from the distanced position to the rest position.

Preferably, the external structure 2 defines a limit stop element 27 for the return spring 53. In other words, the return spring 51 is interposed between the proximal wall of the central portion 51a of the main structure 51 and the limit stop element 27. Preferably, the return spring 53 is fitted around the proximal end 51p of the main structure 51. In other words, the return spring 53 is fitted around the plunger.

The surgical device 1 further comprises a trigger 6 pivoted and solidly joined to the external structure 2. In other words, the external structure 2 comprises, in an upper portion of the external structure 2 itself, a pin 28 to which the trigger 6 is connected and free to rotate, at least by a preset arc. In other words, the trigger 6 is provided with a through hole 62 at one end thereof, into which the pin 28 is inserted.

The trigger 6 is engaged in the main structure 51 of the actuation mechanism 5. In other words, the central portion 51a of the main structure 51 defines a housing seat 57 configured to accommodate the trigger 5. In particular, the external structure 2 has a respective housing 29, in which the pin 28 is present, and inside which the trigger 6 is free to move. The housing 29 is at least partly passed through by the guide 25, inside which the main structure 51 is free to move. Therefore, the housing seat 57 at least partly defines a continuation of the housing 29 and, during use, is positioned over the latter to enable a movement of the trigger 6. Preferably, the housing seat 57 has an angled extension with respect to a reference axis which is parallel, during use, to the main axis of extension "X" of the external element 3.

The trigger 6 is movable between a position distal to the handle 21 and a position proximal to the handle 21.

In the position distal to the handle 21, the main structure 51 is in the rest position and the forceps are in an open configuration.

In the position proximal to the handle 21, the main structure 51 is in the distanced position so as to pull the wire 52 and bring about a closed configuration of the forceps 4.

Preferably, the trigger 6 extends along a main axis of extension thereof which, when the trigger 6 is in the position distal to the handle 21, is substantially parallel to a main axis of extension of the housing seat 57. The main structure 51 further comprises a pin 58 and the trigger 6 has a through hole 61 shaped like a cam, i.e. a shaped cam disposed in a central portion of the trigger 6 itself.

The through hole 61 is pivoted on the pin 58 of the main structure of the actuation mechanism 5.

The pin 58 is preferably defined in the central portion 51a defining the housing seat 57
Advantageously, the through hole 61 shaped like a cam ensures that the trigger 6 cannot in any way exert forces on the actuation mechanism 5 during a movement thereof from the position distal to the handle 21 to the position proximal to the handle 21. In other words, the cam (or shaped cam) ensures a first path of the trigger 6 in which the latter does not move the main structure 51 and thereby risk causing movements different from those "parallel" to the main axis of extension "X".

In other words, the cam or shaped cam ensures a first movement in which the trigger does not interfere with the main structure 51, i.e. the pin 58, and a second movement in which the trigger draws the pin 58 to move the main structure from the rest position to the distanced position.

Therefore, the combination of the through hole 61 and pin 58 makes it possible to obtain a linear movement of the main structure 51 so that the latter does not encounter obstacles and, at the same time, by virtue of the return spring 53, has a facilitated return.

In other words, the present invention allows the main structure 51 to be maintained on axis, which enables an effective opening and closing of the forceps 4.

During use, a user who intends to use the surgical device 1 will insert the latter into the cavity and, upon reaching the portion or fragment of tissue to be removed, will press the trigger 6, which will pass from the position distal to the handle 21 to the position proximal to the handle 21.

During this movement, the actuation mechanism 5 will allow the forceps 4 to pass from the open configuration to the closed position. In particular, the main structure 51 will slide inside the respective seat 25, thereby compressing the return spring 53 and drawing the actuating wire 52. During this movement of the trigger 6, the through hole 61 shaped like a cam or shaped cam ensures that it will not enter immediately in contact with the pin 58, in order to maintain the main structure 51 on axis with respect to the seat 25.

During this movement, the actuating wire 52 draws with it the connecting element 41, bringing the latter from the first position to the second position so as to obtain a drawing of the pair of connecting rods 44a and 44b, which causes a closing of the grasping elements 42a and 42b.

During the actuation of the actuation mechanism 5 by means of the trigger 6, one does not obtain any unwanted transmissions of force that could lead to misalignments and therefore to blocking or obstacles to the closing of the forceps 4.

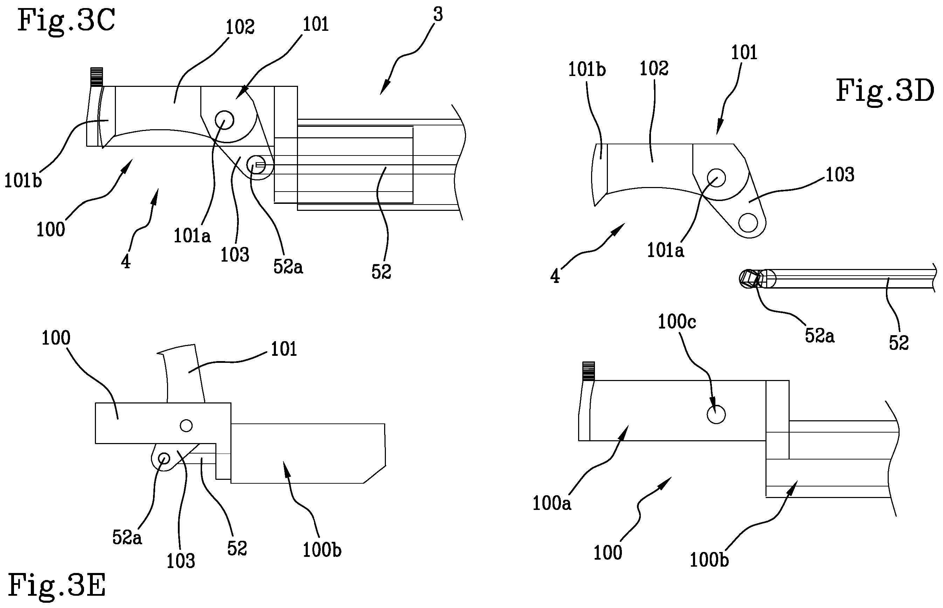

According to an alternative embodiment of the forceps 4, represented in figures 3C, 3D and 3E, the latter comprise a fixed portion 100 and a movable portion 101.

The fixed portion 100 is connected, i.e. defined on the second end 32 of the external element 3.

Preferably, the fixed portion 100 defines a containment volume. Even more preferably, the fixed portion 100 is defined by a main body 100a defining the aforesaid containment volume and a fastening body 100b connected or connectable to the second end 32 of the external element 3. Preferably, the fastening body 100b is fitted over the second end 32 or inserted inside the latter.

The movable portion 101 is pivoted in the fixed portion 100 and has a grasping end 102 and an end 103 connected to the first end 52a of the actuating wire 52. Preferably, the portion movable 101 has a pin 101a pivoted in a respective hole 100c defined in the fixed portion 100. Preferably, the hole 100c is defined in the main body 100a. In other words, the movable portion 101 is able to rotate, by means of the pin 101c, about a rotation axis defined by the hole 100c.

Preferably, the movable portion 101 is made in the form of a blade or has a cutting end 101b.

Therefore, the movable portion 101, during use, is movable, by means of an actuation of the actuation mechanism 5, relative to the fixed portion 100 between a first position and a second position. In other words, the movable portion 101 is able to rotate relative to the fixed portion 100 between the two aforesaid positions.

In the first position, the grasping end 102 is in an open configuration in which it is distanced from the fixed portion 100.

In the second position, the end 103 is distanced from the second end 32 of the external element 3 so as to cause a rotation of the grasping end 102 in order that the latter is brought close to the fixed portion 100 so as to define the closed configuration.

If the fixed portion 100 defines a containment volume (i.e. the main body 100a defines the aforesaid containment volume), in the closing portion the grasping end 102 is nested or pulled close in the containment volume. Advantageously, the fixed portion 100 makes it possible to ensure that the portion or fragment of tissue remains contained inside the fixed portion 100 itself so that the portion or fragment may be extracted without damage that might compromise the subsequent analysis.

From an operating viewpoint, the two embodiments operate in the same manner. An actuation of the actuation mechanism 5 causes a movement of the actuating wire 52, which draws along with it the end 103; this causes the rotation of the grasping end 102, thereby bringing about the closed configuration for retaining and/or removing the portion or fragment of tissue.

Other embodiments of the forceps 4 are possible, all with the intention of pinching, retaining and removing the portion or fragment of tissue to be analysed.

Advantageously, the present invention is capable of overcoming the drawbacks emerging from the prior art.

Advantageously, unwanted blocking of the actuation mechanism 5 is prevented in the surgical device 1.

Advantageously, in the surgical device 1 it is moreover possible to easily adjust an orientation of the forceps 4.

## Claims

1. A surgical device (1) for biopsy comprising:
- an external structure (2) having a handle (21), which is disposed in a proximal portion (2p) of the external structure (2) and may be gripped by a user;
- an external element (3) extending along a main axis of extension (X) between a first end (31), connected to a distal portion (2d) of the external structure (2), and a second end (32), said external element (3) being insertable, during use, into a cavity;
- biopsy forceps (4) retained by said second end (32) of said external element (3);
- an actuation mechanism (5) for said forceps (4), disposed inside said external structure (2), and comprising:
- a main structure (51) movable inside said external structure (2) between a rest position in which the main structure (51) is proximal to said external element (3) and a distanced position in which said main structure (51) is distal relative to said external element (3);
- an actuating wire (52) extending at least partly inside said external element (3) and having a first end (52a) connected to said forceps (4) and a second end (52b) connected to a distal end (51d) of said main structure (51);
- a return spring (53) connected to a proximal end (51p) of said main structure (51) and configured to bring said main structure (51) back from the distanced position to the rest position;
- a trigger (6) pivoted and solidly joined to the external structure (2) and engaged in said main structure (51) of the actuation mechanism (5), said trigger (6) being movable between a position distal to said handle (21), in which said main structure (51) is in said rest position and said forceps (4) are in an open configuration, and a position proximal to said handle (21) in which said main structure (51) is in the distanced position so as to pull said actuating wire (52) and bring about a closed configuration of said forceps (4),
**characterised in that** said main structure (51) comprises a pin (58) and said trigger (6) has a through hole (61) shaped like a cam and pivoted on said pin (58) of the main structure of the actuation mechanism (5).

2. The surgical device (1) according to claim 1, wherein said main structure (51) comprises a central portion (51a) defining a housing seat (57) having said pin (58) and configured to accommodate said trigger (6), said housing seat (57) having angled extension with respect to a reference axis which is parallel, during use, to said main axis of extension (X) of the external element (3).

3. The surgical device (1) according to claim 2, wherein said trigger (6) extends along a main axis of extension thereof which, when said trigger (6) is in said position distal to said handle (21), is parallel to a main axis of extension of said housing seat (57).

4. The surgical device (1) according to one or more of the preceding claims, wherein said proximal end (51p) of the main structure (51) is made in the form of a plunger and said return spring (53) is wound around said plunger, said external structure (2) internally defining a slide guide (26) for said plunger and a limit stop element (27) for said return spring (53).

5. The surgical device (1) according to one or more of the preceding claims, wherein said distal end (51d) of the main structure (51) is made in the form of a chamber (54), preferably cylindrical, said actuation mechanism (5) further comprising a retaining cylinder (56), to which said second end (52b) of the actuating wire (52) is connected, disposed inside said chamber (54) and free to rotate inside said chamber (54).

6. The surgical device (1) according to one or more of the preceding claims, wherein said external element (3) has, at said first end (31), a retaining portion (33) for retaining said external element (3) and wherein said external structure (2) comprises, in said distal portion (2d), a retaining seat (24) for said retaining portion (33).

7. The surgical device (1) according to claim 6, wherein said retaining portion (33) has a cylindrical or discoidal shape and wherein said retaining seat (24) is complementarily shaped with respect to said retaining portion (33), said retaining portion (33) being free to rotate inside said retaining seat (24).

8. The surgical device (1) according to one or more of the preceding claims, wherein said forceps (4) comprise:
- a connecting element (41), disposed inside said external element (3), connected to said first end (52a) of the actuating wire (52) and free to move inside said external element (3) under the action of said actuation mechanism (5);
- a grasping portion (42) defined by two arms pivoted to each other by means of a pin (43) fixed inside said external element (3) and extending between a connecting end (42c) and a grasping end (42a, 42b);
- a pair of connecting rods (44a, 44b), each extending between a first end connected to said connecting element (41) and a second end connected to said connecting end (42c);
wherein said connecting element (41), during use, is movable, by means of an actuation of said actuation mechanism (5), between a first position, in which said connecting element (41) is proximal to said second end (32) of the external element (3) and said grasping portion (42) is in an open configuration wherein the grasping ends (42a, 42b) are distanced from one another, and a second position in which said connecting element (41) is distanced from said second end (32) of the external element (3) so as to draw said pair of connecting rods (44a, 44b) and bring said grasping ends close together (42a, 42b) so as to define said closed configuration.

9. The surgical device (1) according to claim 8, wherein said forceps (4) further comprise, connected to said pin (43) of the grasping portion (42), i.e. to said second end (32) of the external element (3), a tip or needle (45) protruding from said second end (32) of the external element (3).

10. The surgical device (1) according to claim 8 or 9, wherein said grasping ends (42a, 42b) have a cup-like or spoon-like shape so that, in said closed configuration of the forceps (4), they define a containment volume.

11. The surgical device (1) according to one or more of claims 1 to 7, wherein said forceps (4) comprise:
- a fixed portion (100) connected i.e. defined on said second end (32) of said external element (3);
- a movable portion (101) pivoted in said fixed portion and having a grasping end (102) and an end (103) connected to said first end (52a) of the actuating wire (52);
wherein said movable portion (101), during use, is movable, by means of an actuation of said actuation mechanism (5), relative to said fixed portion (100) between a first position, wherein said grasping end (102) is in an open configuration, in which it is distanced from said fixed portion (100), and a second position, in which said end (103) is distanced from said second end (32) of the external element (3) in order to cause a rotation of the grasping end (102) so that it is brought close to the fixed portion (100) so as to define said closed configuration.

12. The surgical device (1) according to claim 11, wherein said fixed portion (100) defines a containment volume inside which said grasping end (102) is nested or pulled close when said forceps (4) are in said closed configuration.
